# EUROPEAN PATENT APPLICATION

(11) **EP 4 737 897 A1**
(43) Date of publication of application: **06.05.2026**
(21) Application number: 24832159.8
(22) Date of filing: 01.07.2024
(51) Int. Cl.: G01N 33/543

(54) **DETECTION MEMBER FOR OBJECT TO BE DETECTED AND METHOD FOR DETECTING OBJECT TO BE DETECTED USING SAME**

(30) Priority: 30.06.2023 US 202363524399 P; 30.06.2023 US 202363524406 P
(71) Applicant: Aoyagi, Tetsuji, Yokohama-shi, Kanagawa 227-0062 (JP)
(72) Inventor: TAKAMURA, Takeji, Atsugi-shi Kanagawa 243-0011 (JP); OTA, Miki, Ayase-shi Kanagawa 252-1126 (JP); AOYAGI, Tetsuji, Yokohama-shi, Kanagawa 227-0062 (JP)
(74) Representative: Swindell & Pearson Limited
(86) International application number: PCT/JP2024/023847
(87) International publication number: WO 2025/005304

(57) **Abstract**

The present invention provides a detection member for an agent to be detected, the detection member including a carrier capable of aggregation on which a DNA aptamer that specifically binds to the agent to be detected is carried, and an antibody that binds to the agent to be detected and has a labeling group that emits light of a specific wavelength upon irradiation with light of a predetermined wavelength, as well as a method for detecting an agent to be detected using the detection member. The agent to be detected may be a virus, a germ, or a bacterium. The virus may be a coronavirus, an influenza virus, a norovirus, or an avian influenza virus.

## Description

### [Technical Field]

The present invention relates to a detection member for an agent to be detected and to a method for detecting an agent to be detected using the detection member.

### [Background Art]

Pathogenic viruses exemplified by an influenza virus and a norovirus are so small that their presence cannot be visually recognized in an ordinary living environment. Such invisibility of viruses increases anxiety and fear in the society and expands the spread of a disease.

In particular, for viruses that are transmitted through the air, the fear due to "being invisible to the eyes" is difficult to overcome. If pathogenic viruses that are present in the air within a living space, an office, a vehicle, and the like, or that adhere to clothing or a mask, could be visualized or detected easily and with high sensitivity without requiring specialized knowledge or operational techniques of biochemical analysis, it would become possible to suppress and prevent viral infection and the spread of a disease.

For the detection of such viruses, in recent years, a relatively simple inspection technique for an influenza virus using immunochromatography, such as the technique disclosed in PTL 1, has been employed.

### [Citation List]

### [Patent Literature]

[PTL 1] Japanese Unexamined Patent Application, Publication No. 2008-275511

### [Summary of Invention]

### [Technical Problem]

A method for detecting an influenza virus using immunochromatography is simple, but for the detection of the virus, it is required that the viruses are present at a concentration of about 10³ to 10⁴ pfu/mL. Therefore, the detection sensitivity at an early stage when the amount of virus present is small is not necessarily high.

In order to suppress infection by pathogens and the spread of a disease, there has been a strong demand for a method that can visualize rapidly and with high sensitivity harmful agents to the human body, such as the viruses that are present in the air within a living space, an office, or a vehicle, or that adhere to clothing or a mask.

The present invention has been made in view of such circumstances, and an object of the present invention is to provide a detection member for an agent to be detected and a method for detecting an agent to be detected using the detection member, which make it possible to visualize the presence of an agent to be detected, within a short time and by a simple technique without using a complicated detection method or a device.

### [Solution to Problem]

To solve the above problem, the detection member for an agent to be detected and the method for detecting an agent to be detected using the detection member according to the present invention employ the following solutions.

A first embodiment of the present invention provides a detection member for an agent to be detected, the detection member including a carrier capable of aggregation and carrying a DNA aptamer that specifically binds to the agent to be detected, and an antibody that binds to the agent to be detected and has a labeling group that emits light of a specific wavelength under a predetermined condition.

In the first embodiment, the agent to be detected may be a virus, a germ, or a bacterium. The virus may be a novel coronavirus, an influenza virus, a norovirus, or an avian influenza virus.

In the first embodiment, the carrier capable of aggregation may be magnetic beads or gold nanoparticles.

In the first embodiment, the antibody having a labeling group that emits light of a specific wavelength under a predetermined condition may be an antibody modified with a fluorescent functional group.

In the first embodiment, the detection member may be an inner sheet of a mask that is provided with a detection patch containing the carrier and the antibody.

In the first embodiment, the detection member may be a wiper that includes a detection reagent containing the carrier and the antibody and that wipes a contacted surface of a detection target that may contains or that the agent to be detected may adhere to.

In the first embodiment, the detection member may be a detection reagent that is sprayed onto a wiper after wiping a contacted surface of a detection target that may contain the agent to be detected or that the agent to be detected may adhere to.

In the first embodiment, the inner sheet of the mask or the wiper may contain moisture at a predetermined ratio.

In the first embodiment, the detection member may be a saliva test stick that is provided with a detection patch containing the carrier and the antibody.

In the first embodiment, the detection member may be a detection display member that is attached to an air conditioner.

In the first embodiment, the detection member may be a detection display member that is attached to a desktop humidifier.

A second embodiment of the present invention provides a method for detecting an agent to be detected using the detection member according to the first embodiment.

In the second embodiment, the method includes a step of bringing the detection member according to the first embodiment into contact with a detection target that may contain the agent to be detected or that the agent to be detected may adhere to, a step of aggregating and collecting the carrier that is contained in the contacted detection member and carrying the DNA aptamer specifically bound to the agent to be detected, a step of bringing the collected carrier having the agent to be detected bound thereto into contact with an antibody that binds to the agent to be detected and is labeled with a labeling group that emits light of a specific wavelength upon irradiation with light of a predetermined wavelength, to form a complex of the agent to be detected and the labeled antibody, and a step of irradiating the formed complex of the agent to be detected and the labeled antibody with light of the predetermined wavelength and detecting the emitted light to detect the agent to be detected.

In the second embodiment, in the step of collecting the aggregated carrier, an instrument having a magnet may be used.

In the second embodiment, in the step of detecting the agent to be detected, light of a predetermined wavelength may be irradiated onto a detection patch that contains the carrier and the antibody and is provided in an inner sheet of a mask.

In the second embodiment, the wiper contains the antibody that binds to the agent to be detected and has a labeling group that emits light of a specific wavelength upon irradiation with light of a predetermined wavelength, and a detection reagent containing the carrier which carries a DNA aptamer that specifically binds to the agent to be detected may be sprayed onto the wiper after wiping a contacted surface of a detection target that may contain the agent to be detected or that the agent to be detected may adhere to, thereby forming the complex of the agent to be detected and the antibody.

### [Advantageous Effects of Invention]

By using the detection member for an agent to be detected according to the present invention, it becomes possible to visualize the presence of a very small amount (subnanomolar) of a virus in a simple manner without using a complicated detection method or a large-scale device that is inconvenient to carry. As a result, detection of a place where viruses are present and measurement of an amount of viruses present can be easily performed even in environments such as a living space and an office.

The detection member for an agent to be detected of the present invention can be designed so that multiple kinds of viruses can be detected by the use of a single detection member. Therefore, it can be applied as an initial screening means for a patient suspected of infection with a virus of which the type cannot be identified only based on a symptom of the patient.

### [Brief Description of Drawings]

[Fig. 1]

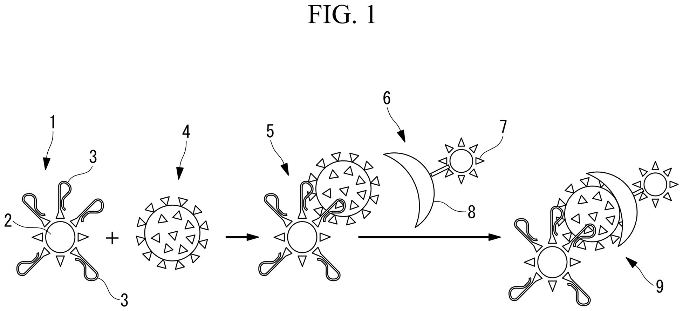

   Fig. 1 is a conceptual diagram showing a mechanism for detecting an agent to be detected using the detection member according to the present invention.
[Fig. 2]

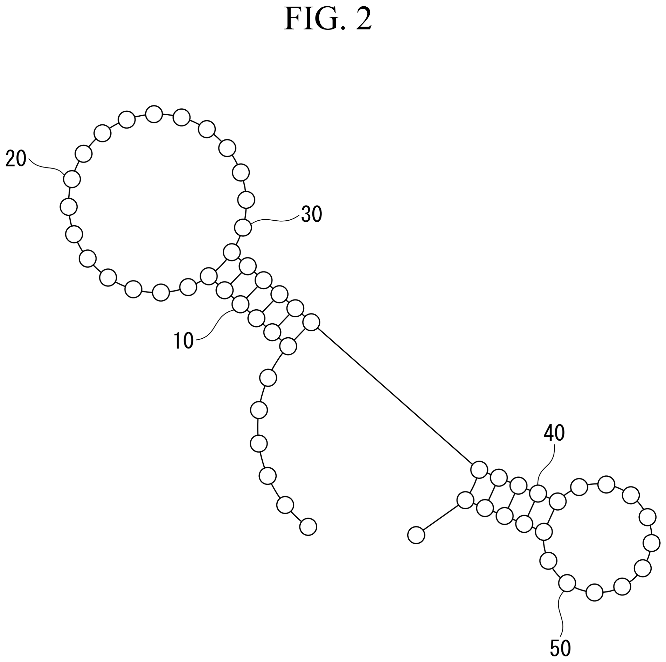

   Fig. 2 is a diagram showing a predicted secondary structure of one example of a DNA aptamer carried on a carrier of the detection member according to the present invention.
[Fig. 3]

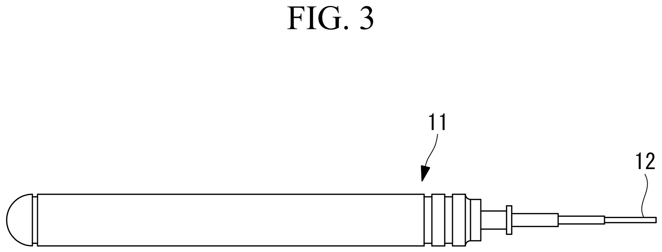

   Fig. 3 is a photograph showing one example of an instrument used for collecting magnetic beads, which is a pen-type instrument having a pen tip that is a magnet.
[Fig. 4]

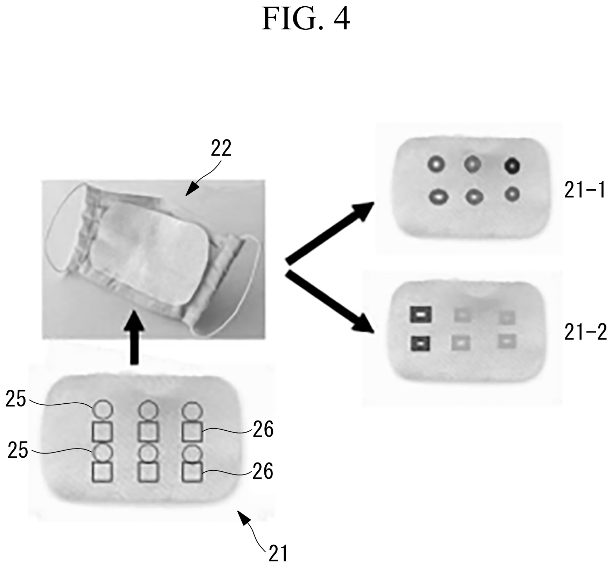

   Fig. 4 is a diagram showing an image of an inner sheet of a mask provided with a detection patch containing a carrier and an antibody, wherein a round patch represents a patch for detecting a coronavirus and a square patch represents a patch for detecting an influenza virus.
[Fig. 5]

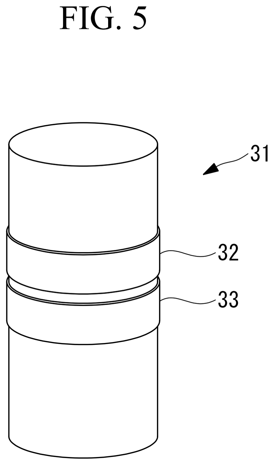

   Fig. 5 is a diagram showing a desktop humidifier provided with a sheet including a detection patch containing the carrier and the antibody of the detection member according to the present invention.
[Fig. 6]

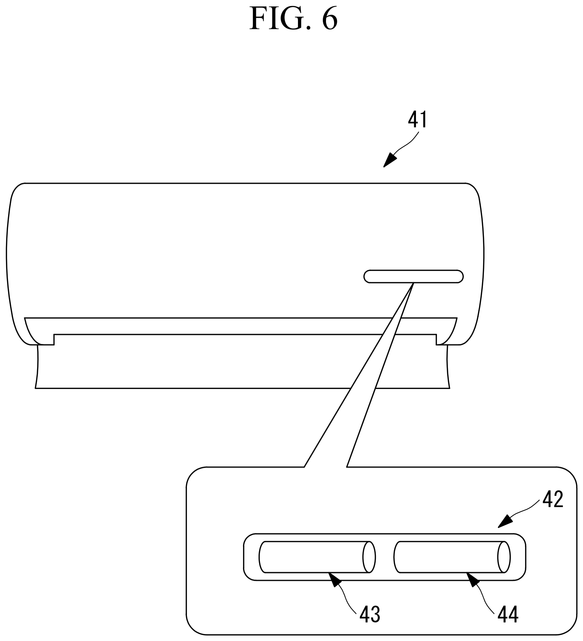

   Fig. 6 is a diagram showing an air conditioner provided with a sheet including a detection patch containing the carrier and the antibody of the detection member according to the present invention.
[Fig. 7]

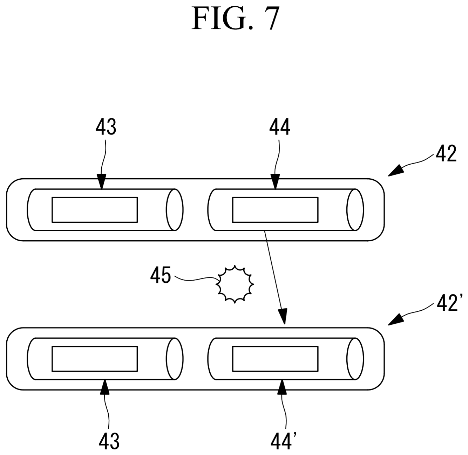

   Fig. 7 is a diagram showing an image of one example of the sheet including a detection patch containing the carrier and the antibody of the detection member according to the present invention, wherein the illustrated example is a sheet includes a patch for detecting a coronavirus and a patch for detecting an influenza virus.
[Fig. 8]

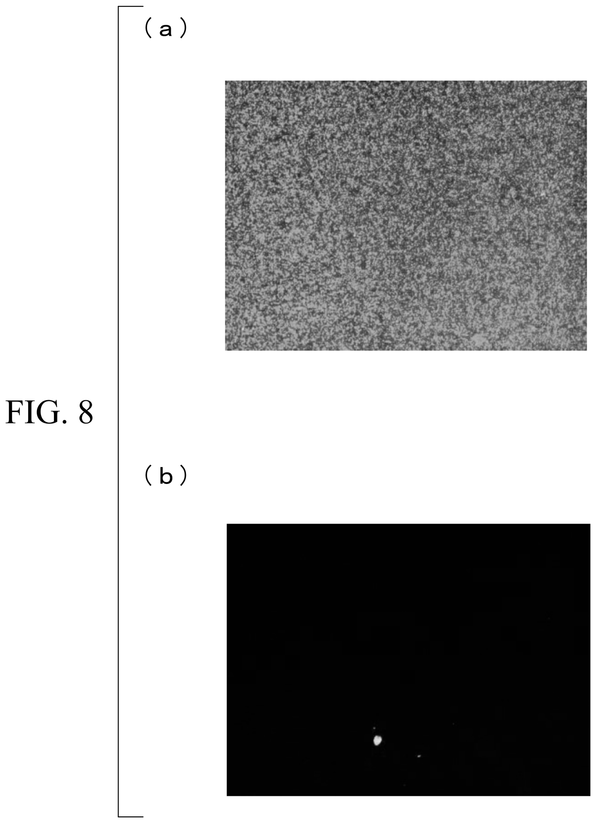

   Fig. 8 is a fluorescent observation image (× 20 magnification) obtained by contacting the detection member according to the present invention with a coronavirus and detecting the resulting complex of the coronavirus and a fluorescently modified antibody, wherein (a) is a fluorescent observation image when a fluorescently modified antibody having spike binding property is used as the antibody, and (b) is a fluorescent observation image when a fluorescently modified antibody without spike binding property is used as the antibody.
[Fig. 9]

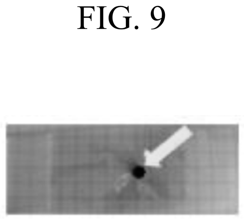

   Fig. 9 is an image showing a state in which magnetic beads applicable as the carrier of the detection member according to the present invention are collected by a magnet attached to a microscope slide.
[Fig. 10]

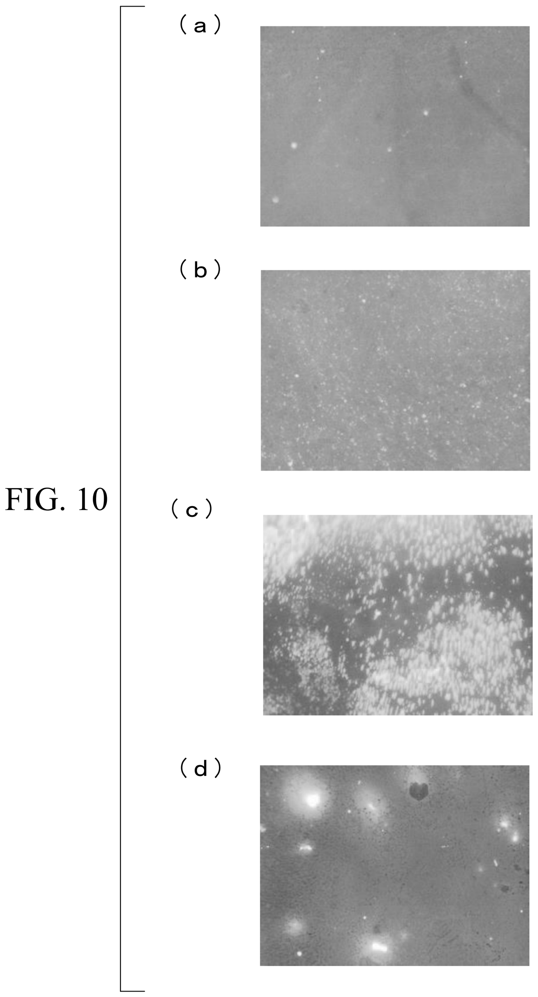

   Fig. 10 is a fluorescent observation image (× 10 magnification) obtained by contacting the detection member according to the present invention with a coronavirus and detecting the resulting complex of the coronavirus and the fluorescently modified antibody, wherein (a) is a fluorescent observation image of a blank, (b) is a fluorescent observation image of 40-fold dilution of a spike protein stock solution, (c) is a fluorescent observation image of the spike protein stock solution, and (d) is a fluorescent observation image after binding of the coronavirus.
[Fig. 11]

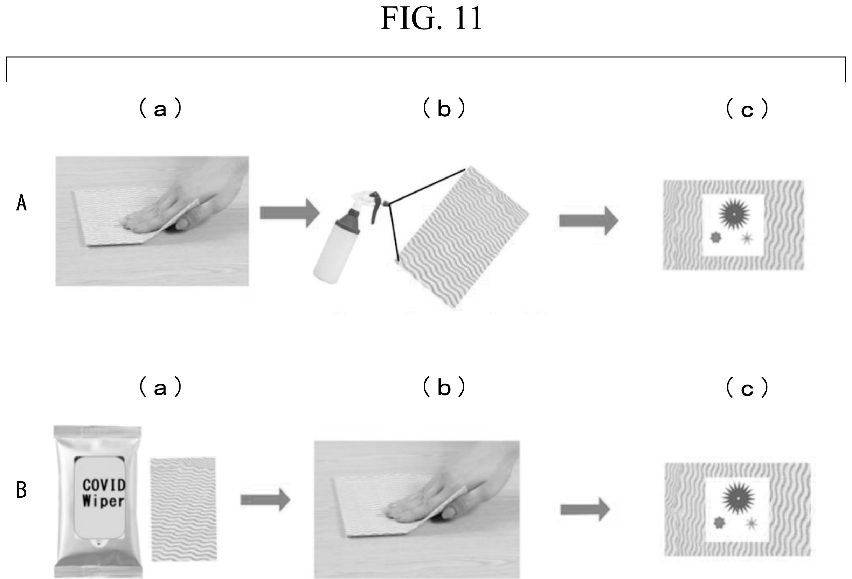

   Fig. 11 is a diagram illustrating an example of use when the detection member according to the present invention is a wiper.

### [Description of Embodiments]

Hereinafter, embodiments of a detection member for an agent to be detected and a method for detecting an agent to be detected using the detection member according to the present invention will be described with reference to the drawings.

In the present embodiments, an aptamer sensor is provided for detecting, with high sensitivity and within a short time such as several seconds to several tens of seconds, a pathogen such as a virus, a germ, and a bacterium by using a simple tool such as a human eye or a camera of a smartphone, without requiring a PCR probe, a large and expensive device, or any operation.

### [First Embodiment]

Hereinafter, a first embodiment of the present invention will be described with reference to Fig. 1. The detection member for an agent to be detected according to the present embodiment includes a carrier capable of aggregation and carrying a DNA aptamer that specifically binds to the agent to be detected, and an antibody that binds to the agent to be detected and has a labeling group that emits light of a specific wavelength under a predetermined condition.

A DNA aptamer is a ligand molecule in which complementary sequences within a DNA molecule form a complementary strand, so that a single-stranded DNA forms a secondary structure or a tertiary structure, and binds specifically and strongly to a target substance by its three-dimensional structure. By binding a DNA aptamer having a specific sequence, the activity of a target molecule can be inhibited, suppressed, or enhanced, or the target molecule can be selectively detected or extracted. The DNA aptamers have recently attracted attention as such molecules.

A DNA aptamer has a smaller molecular weight than that of an antibody, that is about one tenth or less, and yet has high affinity equivalent to that of an antibody and has high target selectivity. The present inventors have found a new approach for detecting an agent to be detected based on a change in fluorescence intensity or a change in fluorescence polarization degree depending on the presence or absence of the agent to be detected by utilizing this high target selectivity.

A DNA aptamer is an artificial DNA obtained by chemical synthesis and having a relatively short chain length (usually 100 bases or less). Since a DNA aptamer can be chemically synthesized, it has advantages such as a low risk of biological contamination, generally low antigenicity, and sufficient stability at a room temperature under conditions near neutral that are free of a nucleic acid degrading enzyme (nuclease) because it is a DNA.

The DNA aptamer can be chemically modified at any position of its sequence with a fluorescent functional group or a fluorescent quencher group in order to create a labeled probe such as a fluorescent probe. Such a high degree of freedom in molecular design is an advantage of using the DNA aptamer that is not present in an antibodies.

The fluorescence polarization method is a method of analyzing the interaction between a fluorescent molecule (here, including a "molecule bound to a fluorescent molecule") and a target substance by measuring fluorescence polarization degree using a fluorescence polarimeter.

The fluorescence polarization measurement has an advantage that it is not necessary to separate a bound fluorescent probe from an unbound fluorescent probe, and a turbid solution can be used as a measurement sample, thereby shortening the time for preparing a sample.

The fluorescence polarization degree refers to the degree of rotation of a fluorescent molecule between the time the fluorescent molecule is excited and the time it emits fluorescence. When a three-dimensional structure of the fluorescent molecule is formed, depending on the three-dimensional structure, the degree of freedom of rotation of the fluorescent molecule may decrease, and the rotation of the fluorescent molecule may become slower. In such a case, the fluorescence polarization degree becomes large. In this way, the movement of the fluorescent molecule is reduced due to the influence of the surrounding stereostructure, or the fluorescent molecule adheres to a large amount of target substance and becomes immobile, that is, fixed. As a result, the rotation of the fluorescent molecule is strictly restricted, and the fluorescence polarization becomes large. By measuring the fluorescence polarization degree, it is possible to rapidly analyze the interaction between the fluorescent molecule and the target substance even with a small amount of sample.

In the present specification, the term "agent to be detected" refers to a substance that can be a specific binding target of a DNA aptamer. The agent to be detected that is detected by using the detection member of the present embodiment includes, for example, a virus, a germ, and a bacterium. Examples of the virus include, but are not limited to, a novel coronavirus (SARS-CoV-2), an influenza virus, a norovirus, and an avian influenza virus.

A method for detecting an agent to be detected will be described using the example shown in Fig. 1. Aptamer beads 1, in which on a carrier 2 capable of aggregation a plurality of DNA aptamers 3 that specifically bind to an agent to be detected are bound, are brought into contact with a detection target that is possibly containing an agent to be detected 4. Examples of the detection target include, but are not limited to, a gas such as air, a liquid that is possibly containing the agent to be detected, and a solid substance such as furniture, equipment, and a medical instrument.

When the detection target contains the agent to be detected 4, the DNA aptamers 3 that specifically bind to the agent to be detected and are bound to the carrier 2 bind to the agent to be detected 4, and an aptamer-agent-to-be-detected complex 5 is formed. Fig. 2 shows a predicted secondary structure of a 57-mer DNA aptamer, which is one example of the DNA aptamer of the present embodiment. The numbers in Fig. 2 indicate the number of bases from the 5' terminal side of the DNA aptamer.

The resulting aptamer-agent-to-be-detected complex 5 is brought into contact with a labeled antibody 6, in which a labeling group 7 that emits light of a specific wavelength under a predetermined condition is labeling an antibody 8 that binds to the agent to be detected. In the present embodiment, a fluorescent functional group or a luminescent functional group can be applied as the labeling group that emits light of a specific wavelength under a predetermined condition.

The antibody 8 that binds to the agent to be detected in the labeled antibody 6 specifically binds to the agent to be detected 4, which is bound to the aptamer-agent-to-be-detected complex 5. As a result, the agent to be detected 4 is bound to both the aggregation-capable carrier 2 and the labeled antibody 6, and thus an aptamer-agent-to-be-detected-labeled antibody complex 9 is formed.

When the formed aptamer-agent-to-be-detected-labeled antibody complex 9 is exposed to a predetermined condition, light of a specific wavelength is emitted. By detecting this light of the specific wavelength, the presence of the agent to be detected is detected. As an example, a case in which a fluorescein isothiocyanate (FITC) group that is a fluorescent functional group is used as a labeling group that emits light of a specific wavelength under a predetermined condition, will be described. In this case, light of 480 nm is irradiated to the aptamer-agent-to-be-detected-labeled antibody complex 9, and thus fluorescence of an amount proportional to the amount of the agent to be detected present is detected at 520 nm. By measuring the amount of this fluorescence, it is possible to measure whether or not the agent to be detected is present and, if present, the amount thereof.

Examples of applicable fluorescent functional groups include fluorescent molecules widely used, such as fluorescein, fluorescein isothiocyanate (FITC), Alexa Fluor (R) 488, Alexa Fluor (R) 514, Texas Red (TM), Cy3 group, 5-carboxytetramethylrhodamine (TAMRA) group, Cy5 group, Cy5.5 group, ROX group, Alexa Fluor (R) 555, and Alexa Fluor (R) 647.

At the first stage of the scheme shown in Fig. 1, that is, when the aptamer beads 1, in which the plurality of DNA aptamers 3 are bound to an aggregation-capable carrier 2, are brought into contact with the detection target that is possibly containing the agent to be detected 4 and thus the complex of the aptamer beads 1 and the agent to be detected 4 is formed, the complex may be collected by utilizing the aggregation ability possessed by the carrier. As the carrier, magnetic beads or gold nanoparticles can be used. When magnetic beads are used, the complex can be easily collected by using an instrument provided with a magnet.

As the magnetic beads, a commercially available product that can be used for separation of a biological substance can be used. The particle size of the magnetic beads is preferably from 50 nm to 5 µm, and more preferably from 0.5 µm to 2 µm. By using an activated functional group on the magnetic beads, a binding molecule such as streptavidin is bound, for example.

As a simpler detection method, the magnetic beads may be collected using a pen-type instrument 11 having a pen tip 12 that is a magnet, as illustrated in Fig. 3. After collecting the magnetic beads with the pen-type instrument 11, if the pen tip 12 emits light under a predetermined condition, the presence or absence of a coronavirus in the treated detection target can be confirmed.

On the other hand, a DNA aptamer designed to specifically bind to the agent to be detected may be modified with a molecule capable of binding to a binding molecule on the magnetic beads. For example, when streptavidin is bound to the magnetic beads, biotin is bound to the DNA aptamer. As a result, the streptavidin on the magnetic bead carrier and the biotin on the DNA aptamer are bound to each other, and the aptamer beads 1 are obtained.

When the binding molecule is bound to the DNA aptamer, the binding molecule is bound to a portion of the DNA aptamer that does not interfere with the binding of the aptamer to the agent to be detected. Preferably, the binding molecule is bound to the 3' or 5' end of the DNA aptamer.

As one example of the detection member of the present embodiment, a fluorescent aptamer-based sensor for specifically detecting a surface proteins of a novel coronavirus and an influenza virus is provided. The aptamer can be designed for specific detection of a wide range of target molecules by utilizing techniques such as SELEX¹. Therefore, the technology according to the present embodiment can be extended to pathogens other than the novel coronavirus and the influenza virus. Since the aptamer is lower in cost, easier to synthesize, and more stable than the antibody², the usefulness of this technology is advantageous compared with the case of using antibodies.

In Example 1 described later, detection of a novel coronavirus was performed using the detection member according to the present embodiment. As a result, the novel coronavirus was successfully detected with high sensitivity.

When the DNA aptamer specifically binds to a target substance, it folds and forms a stable three-dimensional structure. Further, the DNA aptamer can distinguish subtle molecular differences such as those caused by protein mutations³. Due to this high discriminative ability, the agent to be detected can be detected with higher sensitivity even when the agent to be detected is at a low concentration.

Monoclonal antibodies (mAbs) are widely used in biotechnology and in diagnostics such as COVID-19 antigen self-test kits. On the other hand, mAbs have great limitations such as low stability, high production cost, and performance problems including cross-reactions and contamination in proteomic analysis based on mass spectrometry. The detection member according to the present embodiment using a DNA aptamer can avoid the above-described problems.

As a modified example of the detection member or the detection reagent applied to the first embodiment, an aptamer sensor utilizing fluorescence resonance energy transfer (FRET) between two fluorescent molecules can also be used. When FRET occurs, energy is transferred from a donor fluorescent molecule to an acceptor fluorescent molecule by excitation of the donor fluorescent molecule. As a result, the acceptor fluorescent molecule exhibits fluorescence. Therefore, the presence or absence of binding of the agent to be detected to the fluorescently labeled DNA aptamer can be detected as a change in the fluorescence polarization degree at the detection wavelength of the acceptor fluorescent molecule, or as an increase or decrease in the fluorescence intensity.

### [Second Embodiment]

Hereinafter, a second embodiment of the present invention will be described.

As described above, the detection member explained in the first embodiment is brought into contact with the detection target that is possibly containing the agent to be detected 4 by using the aptamer beads 1. The detection target may be a gas such as air, a liquid that is possibly containing the agent to be detected, or a solid substance such as furniture, equipment, or a medical instrument. Therefore, the detection member can take various forms.

Hereinafter, the description will be provided for each specific form of the detection member according to the present embodiment.

### (A) Application to Inner Sheet of Mask

Detection of a novel coronavirus and an influenza virus inside a face mask is performed through an inner sheet containing a fluorescent aptamer sensor.

As one example of the fluorescent aptamer for detecting the novel coronavirus or the influenza virus, a DNA aptamer that binds with high affinity to a receptor binding domain of a spike protein of the novel coronavirus is used. This DNA aptamer has affinity comparable to that of a commercially available antibody that binds to the spike protein.

Mutations of the spike protein of the novel coronavirus, particularly mutations in the receptor binding domain of the spike protein, affect the rate of host cell entry by the virus and generate variants of concern (VoC). The aptamer is required to be designed to recognize the most recent type of coronavirus currently spreading.

In order to enable simultaneous detection of multiple viruses such as the novel coronavirus and the influenza virus, an inner sheet provided with a plurality of types of detection reagents is inserted into a face mask. Viruses detected on the inner sheet are resulting from either the air exhaled by the wearer or from the exposure to the viruses in the external air. In either case, when fluorescence is observed on the inner sheet, it indicates that a detailed diagnostic test is necessary. By using the detection member of the present embodiment as an inner sheet, the face mask becomes a real-time virus detector.

A virus sensor patch, specifically a cellulose filter containing an aptamer sensor to which fluorescent molecules specific to each virus are bound, is printed on a cellulose sheet serving as the base material of an inner sheet 21. By binding different fluorescent molecules, sensors for different viruses such as the novel coronavirus and the influenza virus are distinguished by different colors, as shown in 21-1 and 21-2 of Fig. 4.

In the example of Fig. 4, the images of the inner sheet 21-1 detected when virus A is present and the inner sheet 21-2 detected when virus B is present are shown. If both virus A and virus B are contained in exhaled or inhaled air, detection can be performed by the color change of multiple types of virus sensor patches. Further, as illustrated by reference numerals 25 and 26 in the inner sheet 21 of Fig. 4, the shape of the sensor patch for each target virus can also be varied. The inner sheet 21 is inserted into a mask 22 as shown in Fig. 4.

### (B) Detection Display Member (Indicator) Attached to Air Conditioners, Humidifiers, and Other General Indoor Surfaces

The virus sensor patch described in (A) can be attached to the surface of an air conditioner or a humidifier via an adhesive. By using this sensor patch as an indicator, it is possible to monitor whether the indoor air is contaminated with airborne viruses. As illustrated in Fig. 5 and Fig. 6, the cellulose sheet including the patch can be attached to any surface of equipment via a materials such as double-sided tape.

Fig. 5 shows a desktop humidifier 31 to which detection patch-including sheets 32 and 33 containing the detection member of the present embodiment are attached on the surface. By attaching the detection patches targeted at different viruses simultaneously, detection of the presence or absence of either virus or both viruses can be performed. Detection of the presence or absence of a virus can be observed in a simple manner by irradiating the patch with light of a wavelength at which the labeling group contained in the detection patch emits fluorescence.

Fig. 6 shows an indoor air conditioner 41 in which a base 42 provided with detection patch-including sheets 43 and 44 containing the detection member of the present embodiment is attached to the surface. By attaching the detection patches 43 and 44 targeted at different viruses simultaneously, detection of the presence or absence of either virus or both viruses can be performed. Detection of the presence or absence of a virus can be observed in a simple manner by irradiating the patch with light of a wavelength at which the labeling group contained in the detection patch emits fluorescence. In the example of Fig. 7, since the agent to be detected (virus) 45 targeted by the detection patch 44 is present, the color of the detection patch 44 has changed to form the detection patch 44'. Accordingly, it becomes possible to detect in a simple manner whether or not viruses are floating in the indoor air.

### (C) Saliva Test Instrument

The fluorescent aptamer sensors for the novel coronavirus and the influenza virus are used as a saliva test instrument for detecting the novel coronavirus (COVID-19) and the influenza virus. Compared with the generally used antigen testing kits for the novel coronavirus, a much shorter response time can be achieved, that is, it becomes possible to perform the detection in seconds rather than in minutes.

The DNA aptamer applied to the configuration of this instrument binds to viral surface proteins in the presence of moisture. When detecting viruses in the air, respiratory droplets contain the moisture.

### (D) Application to Wiping Sheet

For detection of viruses on a surface, a wiping sheet that enables wiping, sterilizing, and detecting in a single step is used as the detection member. Examples of the surface to be contacted with the detection target include, but are not limited to, a desk, a seat in a vehicle, and a clothing surface. In surface detection using the wiping sheet, the wiping sheet including an aptamer detection patch is pre-wetted with a reagent solution, or the reagent solution is sprayed onto the sheet after wiping.

By using the wiping sheet, it can be detected in a simple manner whether the virus to be detected is present or absent on the surfaces of a living space, office, a vehicle seat, and clothing. When provided in the form of a wet sheet, it can also be carried.

By using the detection member according to the present embodiment, viruses existing on the surfaces of furniture in a living space, an office space, or a vehicle cabin, or viruses inhaled into a filter inserted into a face mask, can be detected. As a result, the anxiety and the fear in the society caused by the invisibility of viruses can be reduced, and transmission of diseases can be suppressed.

The detection of the SARS-CoV-2 or the influenza virus using a face mask is more efficient and more cost-effective than undergoing an antigen test. By applying the detection member of the present embodiment to the detection of airborne viruses in an office, in a living space, and inside a vehicle, infection in such a space can be avoided, and if viruses are detected, the transmission can be suppressed by prompt decontamination.

### [Examples]

### Example 1: Method for Detecting Coronavirus Using Magnetic Beads

Magnetic beads to which DNA aptamers were bound were prepared, and the magnetic beads were brought into contact with a coronavirus so that the aptamers on the beads bound to the coronavirus. The beads were then collected. A fluorescent antibody having spike binding ability was bound to the coronavirus bound to the collected beads so that a complex was formed, and the fluorescence thereof was measured.

The sequences of the DNA aptamers used in this example are shown below. An aptamer (57-mer) having biotin at the 5' end and an aptamer (54-mer) having biotin at the 3' end were prepared and used independently in the experiments.

**[Table 1]**

| | Sequence (5' to 3') |
|---|---|
| 5'-Biotin Aptamer | 5'-Biotin-TTTTTTCAGCACCGACCTTGTGCTTTGGGAGTGCT- GGTCCAAGGGCGTTAATGGACA |
| 3'-Biotin Aptamer | CAGCACCGACCTTGTGCTTTGGGAGTGCTGGTCCAAGGGC- GTTAATGGACATTT-3'-Biotin |

### Test Method

### 1. Magnetic Beads

In the present Example, the following magnetic beads were used:
Magnetic Beads MyOne C1: Beads obtained by binding streptavidin to superparamagnetic polymer beads (particle size 1.0 µm, concentration 10 mg/mL (7-12×10⁹ beads/mL)) having hydrophilic surfaces and based on carboxyl-activated beads.

### 2. Preparation Method of Aptamer-Bound Magnetic Beads

1) The magnetic bead (MB) solution (20 µL) was washed and dispersed in 200 µL of 2 × BW buffer. (BW: 10 mM Tris-HCl pH 7.5, 1 mM EDTA, 2 M NaCl)
2) 4 µL of the aptamer (100 µM) was diluted with 100 µL of purified water.
3) The MB solution prepared in 1) and the aptamer solution prepared in 2) were mixed, and after standing at a room temperature for 15 minutes, the MBs were collected and redispersed in 200 µL of HEPES (20 mM) (concentration: 200 µg/200 µL).

### 3. Aptamer-Bound Magnetic Beads

**[Table 2]**

| | Concentration |
|---|---|
| Magnetic beads | 1 mg/mL |
| Aptamer | 400 pmol/mg beads (theoretical value) |

It was confirmed that 8 pmol of aptamer was present in 20 µL of aptamer-bound magnetic beads.

### Binding of Coronavirus to Magnetic Beads and Subsequent Binding with Fluorescent Antibody

(1) To 20 µL of the aptamer-bound magnetic beads, 5 µL of heat-inactivated coronavirus dispersion (SARS-related Coronavirus 2 culture fluid, ZeptoMetrix, at an unknown concentration) or Spike solution (SARS-CoV-2 (2019-nCoV) Spike S1 (D614G)-His Recombinant Protein, HPLC-verified, Shino Biological, 0.25 mg/mL) was added, shaken for 20 minutes at a room temperature, and allowed to bind. The magnetic beads were collected using a magnet and washed twice with 100 µL of HEPES (20 mM).
(2) The obtained complex of the aptamer-bound magnetic bead and the coronavirus was dispersed in 100 µL of HEPES, and 5 µL of Anti-Spike Antibody (SARS-CoV-2 Spike Protein (RBD) Recombinant Human Monoclonal Antibody (P05DHu), Alexa Fluor (R) 488, eBioscience, 0.5 mg/mL) was added, and the solution was shaken for 30 minutes at a room temperature.
(3) The magnetic beads were collected, washed with HEPES, dispersed in 200 µL of HEPES, and the fluorescence was measured.

Table 3 shows the measurement results of the fluorescence detection by the obtained aptamer-captured spike antibody complex. In Table 3, "blank" indicates the value of a sample not containing the spike protein or the coronavirus.

**[Table 3]**

| | 3'-Biotin Aptamer | Blank | 5'-Biotin Aptamer | Blank |
|---|---|---|---|---|
| Fluorescence count | 43229 | 2543 | 53012 | 2943 |

Fig. 8 shows a fluorescence observation image (×20) obtained by dispersing the sample on black filter paper.

### Improvement of Sensitivity by Collection Using Magnet

By using a neodymium magnet with a diameter of 3 mm to collect the magnetic beads, the magnetic beads (coronavirus) were concentrated. By aggregating the magnetic beads with a magnet, it was confirmed that the detection at a locally high concentration was possible, as shown in Fig. 9.

Fig. 10 shows a fluorescence observation image (×10) obtained. The four images in Fig. 10 are fluorescence observation images (×10 magnification), in which (a) is an image of a blank, (b) is an image of a 40-fold diluted solution of the spike protein stock solution, (c) is an image of the spike protein stock solution, and (d) is an image after coronavirus binding, in which the aptamers were brought into contact with the coronavirus and the obtained complex of the coronavirus and the fluorescently modified antibody was detected. The present Example confirmed that virus can be detected using a desktop-sized measurement device without requiring a large-scale and expensive measuring equipment.

### Example 2: Surface Detection Using Wiper

A wiper that enables wiping, sterilization, and detection in a single step is prepared. A cellulose sheet including a virus detection patch is used as the wiper. The wiper may take two forms, (A) and (B), described below.
(A) An example of the form in which a cellulose sheet including a virus detection patch is used is shown in Fig. 11A. As shown in (a), the surface of an object on which a virus may be present is wiped using the cellulose sheet including the virus detection patch. Next, as shown in (b), a solution containing a detection reagent is sprayed onto the sheet after wiping. Finally, as shown in (c), light of a predetermined wavelength is irradiated onto the sheet sprayed with the detection reagent, and the presence or absence of the virus is detected based on the presence or absence of fluorescence or color development on the sheet.
(B) An example of the form in which the cellulose sheet including a virus detection patch is pre-wetted with a solution containing a detection reagent is shown in Fig. 11B. As shown in (a), the cellulose sheet including the virus detection patch is pre-wetted with the solution containing the detection reagent, and is stored as a wet sheet. Next, as shown in (b), the surface of an object on which a virus may be present is wiped using the sheet impregnated with the detection reagent. Finally, as shown in (c), light of a predetermined wavelength is irradiated onto the sheet impregnated with the detection reagent, and the presence or absence of the virus is detected based on the presence or absence of fluorescence or color development on the sheet.

As the detection member or the detection reagent applied in the Example 2, an aptamer sensor utilizing fluorescence resonance energy transfer (FRET) between two fluorescent molecules may also be used. When FRET occurs, energy is transferred from the donor fluorescent molecule to the acceptor fluorescent molecule upon excitation of the donor fluorescent molecule. As a result, the acceptor fluorescent molecule emits fluorescence. Accordingly, the presence or absence of binding of an agent to be detected to the fluorescently labeled DNA aptamer can be detected as a change in the fluorescence polarization degree or an increase in the fluorescence intensity at the detection wavelength of the acceptor fluorescent molecule.

### (References)

1. Sefah, K., Shangguan, D., Xiong, X. et al., Development of DNA aptamers using Cell-SELEX. Nat Protoc 5: 1169-1185 (2010).
2. Thiviyanathan, V., Gorenstein, DG. Aptamers and the next generation of diagnostic reagents. Proteomics Clin Appl 6: 563-573 (2012).
3. Conrad, R., Ellington, AD. Detecting immobilized protein kinase C isozymes with RNA aptamers. Anal Biochem. 242: 261-265 (1996).

### [Reference Signs List]

- 1: Aptamer beads
- 2: Carrier
- 3: DNA aptamer
- 4: Agent to be detected
- 5: Aptamer-agent-to-be-detected complex
- 6: Labeled antibody
- 7: Labeling group
- 8: Antibody binding to agent to be detected
- 9: Aptamer-agent-to-be-detected-labeled antibody complex
- 11: Pen-type instrument
- 12: Pen tip
- 21: Inner sheet
- 21-1: Inner sheet when virus A is present
- 21-2: Inner sheet when virus B is present
- 22: Mask
- 25: Sensor patch
- 26: Sensor patch
- 31: Humidifier
- 32: Detection patch-including sheet
- 33: Detection patch-including sheet
- 41: Air conditioner
- 42: Base
- 42': Base after use
- 43: Detection patch-including sheet
- 44: Detection patch-including sheet
- 44': Sheet after detection of agent to be detected
- 45: Agent to be detected

## Claims

1. A detection member for an agent to be detected, comprising:
a carrier capable of aggregation and carrying a DNA aptamer that specifically binds to the agent to be detected; and
an antibody that binds to the agent to be detected and has a labeling group that emits light of a specific wavelength under a predetermined condition.

2. The detection member for an agent to be detected according to Claim 1, wherein the agent to be detected is a virus, a germ, or a bacterium.

3. The detection member for an agent to be detected according to Claim 2, wherein the virus is a coronavirus, an influenza virus, a norovirus, or an avian influenza virus.

4. The detection member for an agent to be detected according to Claim 1, wherein the carrier capable of aggregation is magnetic beads or gold nanoparticles.

5. The detection member for an agent to be detected according to Claim 1, wherein the antibody having a labeling group that emits light of a specific wavelength upon irradiation with light of a predetermined wavelength is an antibody modified with a fluorescent functional group.

6. The detection member for an agent to be detected according to Claim 1, wherein the detection member is an inner sheet of a mask that is provided with a detection patch containing the carrier and the antibody.

7. The detection member for an agent to be detected according to Claim 1, wherein the detection member is a wiper that includes a detection reagent containing the carrier and the antibody and that wipes a contacted surface of a detection target that may contain the agent to be detected or that the agent to be detected may adhere to.

8. The detection member for an agent to be detected according to Claim 1, wherein the detection member is a detection reagent that contains the carrier and the antibody and that is sprayed onto a wiper after wiping a contacted surface of a detection target that may contain the agent to be detected or that the agent to be detected may adhere to.

9. The detection member for an agent to be detected according to Claim 1, wherein the detection member is a saliva test stick that is provided with a detection patch containing the carrier and the antibody.

10. The detection member for an agent to be detected according to Claim 1, wherein the detection member is a detection display member that is attached to an air conditioner.

11. The detection member for an agent to be detected according to Claim 1, wherein the detection member is a detection display member that is attached to a desktop humidifier.

12. A method for detecting an agent to be detected using a detection member for an agent to be detected comprising:
a carrier capable of aggregation and carrying a DNA aptamer that specifically binds to the agent to be detected; and
an antibody that binds to the agent to be detected and has a labeling group that emits light of a specific wavelength under a predetermined condition,
the method comprising:
a step of bringing the detection member into contact with a detection target that may contain the agent to be detected or that the agent to be detected may adhere to;
a step of aggregating and collecting the carrier that is contained in the contacted detection member and carrying the DNA aptamer specifically bound to the agent to be detected;
a step of bringing the collected carrier having the agent to be detected bound thereto into contact with an antibody that binds to the agent to be detected and is labeled with a labeling group that emits light of a specific wavelength upon irradiation with light of a predetermined wavelength, to form a complex of the agent to be detected and the labeled antibody; and
a step of irradiating the formed complex of the agent to be detected and the labeled antibody with light of a predetermined wavelength and detecting the emitted light to detect the agent to be detected.

13. The method for detecting an agent to be detected according to Claim 12, wherein in the step of collecting the aggregated carrier, the carrier is collected using an instrument having a magnet.

14. The method for detecting an agent to be detected according to Claim 12, wherein in the step of detecting the agent to be detected, light of a predetermined wavelength is irradiated onto a detection patch that contains the carrier and the antibody and is provided in an inner sheet of a mask.

15. The method for detecting an agent to be detected according to Claim 12, wherein in the step of forming the complex of the agent to be detected and the antibody, the wiper contains the antibody, and a detection reagent containing a carrier which carries a DNA aptamer that specifically binds to the agent to be detected is sprayed onto the wiper after wiping a contacted surface of a detection target that may contain the agent to be detected or that the agent to be detected adhere to, thereby forming the complex of the agent to be detected and the antibody.
